# EUROPEAN PATENT SPECIFICATION

(11) **EP 3 815 729 B1**
(45) Date of publication and mention of the grant of the patent: **05.08.2026**
(21) Application number: 18923921.3
(22) Date of filing: 26.06.2018
(51) Int. Cl.: A61M 16/00, A61M 16/01, A61M 16/10, A61M 16/12

(54) **ANESTHESIA MACHINE AND SYSTEM**
ANÄSTHESIEGERÄT UND SYSTEM
MACHINE D'ANESTHÉSIE ET SYSTÈME

(43) Date of publication of application: 05.05.2021
(73) Proprietor: Shenzhen Mindray Bio-Medical Electronics Co., Ltd., Shenzhen, Guangdong 518057 (CN); Shenzhen Mindray Scientific Co., Ltd., Shenzhen, Guangdong 518000 (CN)
(72) Inventor: HUANG, Chenghua, Shenzhen, Guangdong 518057 (CN); PAN, Ruiling, Shenzhen, Guangdong 518057 (CN); LIANG, Dongsheng, Shenzhen, Guangdong 518057 (CN); HUANG, Jiping, Shenzhen, Guangdong 518057 (CN)
(74) Representative: KIPA AB
(86) International application number: PCT/CN2018/092937
(87) International publication number: WO 2020/000208

(56) References cited:
- WO-A1-2016/133406
- WO-A1-2016/157102
- CN-A- 103 809 619
- CN-A- 106 964 045
- CN-U- 201 840 744
- CN-U- 204 671 697
- CN-Y- 201 058 168
- US-A1- 2010 175 695
- US-A1- 2010 175 695
- US-A1- 2019 358 418

## Description

### CROSS-REFERENVE TO RELATED APPLICATIONS

This application is a continuation of PCT Application NO. PCT/CN2018/092937, filed Jun. 26,2018, entitled "ANESTHESIA MACHINE AND SYSTEM".

### TECHNICAL FIELD

The present application relates to the technical field of medical devices, and in particular to an anesthesia machine and system.

### BACKGROUND

An anesthesia machine functions to perform inhalation anesthesia and mechanical ventilation for a patient during surgery. With the help of an anesthesia ventilator, the patient's airway is maintained open, and the ventilation and oxygenation are improved, thereby preventing the body from hypoxia and CO₂ accumulation. However, during an operation with general anesthesia, the patient usually needs to be subjected to ventilation by intubation. Therefore, it is necessary to extend the asphyxia time window of the patient to reserve time for intubation by a surgeon. In general, this can be achieved by means of pre-oxygenation to the patient.

US 2010/175695 A1 discloses an anesthesia system which includes a main output and an auxiliary output. The main output and the auxiliary output are configured to operate independently of each other. The main output may provide a first mixture of gases and an anesthetic agent to a patient. The auxiliary output may provide a second mixture of gases to the patient. In certain embodiments, a user may selectively mix oxygen and at least one other gas, such that the second mixture of gases is provided to the auxiliary output at a desired flow rate and includes a desired oxygen percentage level. The second mixture of gases provided through the auxiliary output is prevented from flowing back into the anesthesia system to reduce or prevent contamination of the gases provided through the main output.

WO 2016/157102 A1 discloses an apparatus for promoting gas exchange (such as oxygenation and/or CO2 removal) to provide, in one application, respiratory support for a patient, in relation to anaesthesia or more generally medical procedures where respiratory function might be compromised.

WO 2016/133406 A1 discloses a system for oxygenating a patient in relation to anaesthesia using high flow gas delivery. The system has a flow source, and a controller for determining oxygenation requirements of the patient before or during anaesthesia.

### SUMMARY

The invention is defined in the appended independent claims 1 and 9. Preferred embodiments are matter of the dependent claims.

According to a first aspect of the present application, the present application provides an anesthesia machine including a gas source interface, and an oxygen supply apparatus and an anesthesia breathing apparatus respectively connected to the gas source interface, wherein the oxygen supply apparatus is configured to provide oxygen-containing gas to a patient; and the anesthesia breathing apparatus is configured to provide anesthesia breathing support to the patient.

According to a second aspect of the present application, the present application provides an anesthesia machine system including an anesthesia machine and an oxygen supply device, wherein the anesthesia machine is provided with a fixing mechanism, and the oxygen supply device **is** detachably fixed to the anesthesia machine by means of the fixing mechanism.

### BRIEF DESCRIPTION OF THE DRAWINGS

In order to more clearly illustrate technical solutions in the embodiments of the present application or in the prior art, a brief introduction to the drawings required for the description of the embodiments or the prior art will be provided below. Obviously, the drawings in the following description are only some of the embodiments of the present application, and those of ordinary skill in the art would have also been able to obtain other drawings from these drawings without involving any inventive effort.
FIG. 1a is a schematic structural diagram of an anesthesia machine according to an embodiment;
FIG. 1b is a schematic structural diagram of an anesthesia machine according to an embodiment;
FIG. 1c is a schematic structural diagram of an anesthesia machine according to an embodiment;
FIG. 2a is a schematic structural diagram of an oxygen supply apparatus of an anesthesia machine according to an embodiment;
FIG. 2b is a diagram of a gas path of an oxygen supply apparatus of an anesthesia machine according to an embodiment;
FIG. 2c is a schematic structural diagram of an oxygen supply apparatus of an anesthesia machine according to an embodiment;
FIG. 3a is a specific structural diagram of an implementation solution of an oxygen supply apparatus of an anesthesia machine according to an embodiment;
FIG. 3b is a specific structural diagram of an implementation solution of an oxygen supply apparatus of an anesthesia machine according to an embodiment;
FIG. 4 is a schematic structural diagram of an anesthesia breathing apparatus of an anesthesia machine according to an embodiment;
FIG. 5 is a structural diagram of an implementation solution of a breathing control module and a breathing circuit in an anesthesia breathing apparatus of an anesthesia machine according to an embodiment;
FIG. 6 is a schematic structural diagram of an anesthesia machine according to an embodiment;
FIG. 7a is a schematic perspective structural diagram of an anesthesia machine according to an embodiment; and
FIG. 7b is a schematic partial enlarged diagram of an anesthesia machine according to an embodiment.

### DESCRIPTION OF THE PREFERRED EMBODIMENTS

Before the embodiments of the present application are described in detail, the pre-oxygenation technology is briefly introduced. The pre-oxygenation technology generally may refer to the use of high-concentration oxygen inhalation before anesthesia induction and tracheal intubation to delay the decrease of arterial oxygen saturation caused by apnea. Of course, the oxygen supply apparatus, the oxygen supply device, etc. for providing oxygen-containing gas mentioned in the present application may be not limited to the application to the foregoing pre-oxygenation technology, but can also be applied to other clinical scenarios requiring oxygen supply, such as postoperative oxygen supply.

In general, during the preoperative pre-oxygenation and intubation, a separate oxygen supply device may be used to supply oxygen to a patient. For a patient with difficult airway, the asphyxia time window can be extended to provide time for a surgeon to establish artificial airway. However, at present, it may be necessary to use a separate oxygen supply device during oxygen supply to a patient, which may increase the difficulty of device management in a tense surgical environment. There may also be a risk of insufficient resources or failure to find the machine in an emergency. Moreover, the information of the current oxygen supply device itself may be too independent to be shared to an anesthesia machine and other information systems.

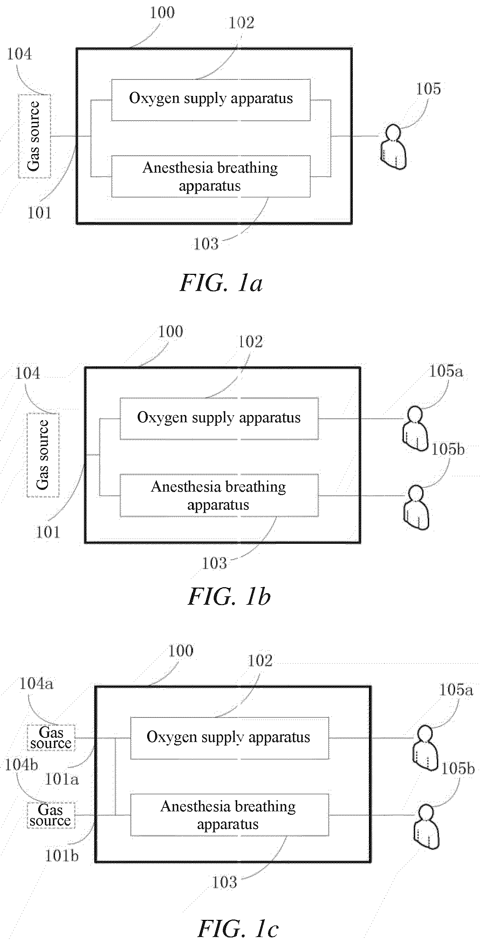

Based on the above factors, the present application provides an anesthesia machine. As shown in FIGS.1a and 1b, the anesthesia machine 100 may include a gas source interface 101, and an oxygen supply apparatus 102 and an anesthesia breathing apparatus 103 which may be respectively connected to the gas source interface 101. The oxygen supply apparatus 102 may be configured to provide oxygen-containing gas to a patient 105. The anesthesia breathing apparatus 103 may be configured to provide anesthesia breathing support to the patient 105.

The gas source interface 101 may be connected to an external gas source 104 to provide desired gas, may include oxygen, laughing gas (nitrous oxide), air, etc., to the anesthesia machine. In some cases, the gas provided by the gas source may be high-pressure gas, such that a gas outlet of the gas source may be provided with a pressure reducing valve, or a pressure reducing valve may be provided at the gas source interface 101 of the anesthesia machine, so as to provide stable gas to the anesthesia machine.

In addition to the anesthesia breathing apparatus 103 for providing anesthesia breathing support to the patient, the anesthesia machine provided by the present application may further integrate the oxygen supply apparatus 102, such that the anesthesia machine can be directly used to perform oxygen supply operation for the patient, so as to extend the asphyxia time window of the patient to reserve time for other operations (such as intubation) by a surgeon.

In some embodiments, as shown in FIG. 1a, the anesthesia machine may include only one gas source interface 101. The gas source interface 101 may be connected to an external gas source 104 to provide oxygen to the anesthesia machine. Oxygen input through the gas source interface 101 respectively may enter the gas paths of the oxygen supply apparatus 102 and/or the anesthesia ventilator apparatus 103 according to a working state of the anesthesia machine, and may be finally delivered to the patient 105. The oxygen supply apparatus 102 may provide the oxygen-containing gas to the patient 105, and the anesthesia ventilator apparatus 103 may provide anesthesia breathing support to the patient 105.

As shown in FIG. 1a, the oxygen supply apparatus 102 and the anesthesia breathing apparatus 103 may share one output interface, for example, they may share one mask for the patient 105 to use. In these embodiments, due to the limitation of only one output interface, only one of the oxygen supply apparatus 102 and the anesthesia breathing apparatus 103 may be in the working state. Specifically, the oxygen supply apparatus 102 may share an ACGO outlet (auxiliary fresh gas outlet), a respiratory system inhalation port or other outlets of the anesthesia breathing apparatus.

In other embodiments, as shown in FIG. 1b, the oxygen supply apparatus 102 and the anesthesia breathing apparatus 103 may respectively have independent gas outlets, for example, they may be provided with separate masks. Of course, in these embodiments, the oxygen supply apparatus 102 and the anesthesia breathing apparatus 103 can be independently operated and respectively used by a patient 105a and a patient 105b. Specifically, the oxygen supply apparatus 102 and the anesthesia breathing apparatus 103 can be equipped with independent starting switches and controlled independently; or the anesthesia machine can be equipped with a main switch so as to control, through a software control interface of the anesthesia machine, when the oxygen supply apparatus 102 and the anesthesia breathing apparatus 103 may need to be independently turned on or off. Of course, the software control interface of the anesthesia machine can also be configured to respectively control other functions and states of the oxygen supply apparatus 102 and the anesthesia breathing apparatus 103.

In general, when performing oxygen supply operation and anesthesia breathing support for a patient, the anesthesia machine may not only use oxygen as the source gas, but also may need to use equilibrium gas, such as laughing gas and air, so as to achieve the purpose of regulating the concentration of oxygen. Based on this, as shown in FIG. 1c, in some embodiments, the anesthesia machine may be different from that shown in FIG. 1b in that the gas source interface may include an oxygen input interface 101a for providing oxygen and an equilibrium gas input interface 101b for providing equilibrium gas. The oxygen input interface 101a may be connected to an external oxygen gas source 104a, and the equilibrium gas input interface 101b may be connected to an external equilibrium gas source 104b. Of course, in other embodiments, the anesthesia machine can provide more than two gas source interfaces to facilitate providing more than two gases in number to the anesthesia machine.

The anesthesia machine structure solution shown in FIG. 1c will be described in detail below, and some contents in these descriptions can be applied to the anesthesia machine structure solution shown in FIGS. 1a and 1b based on the knowledge acquired by those of ordinary skill in the art.

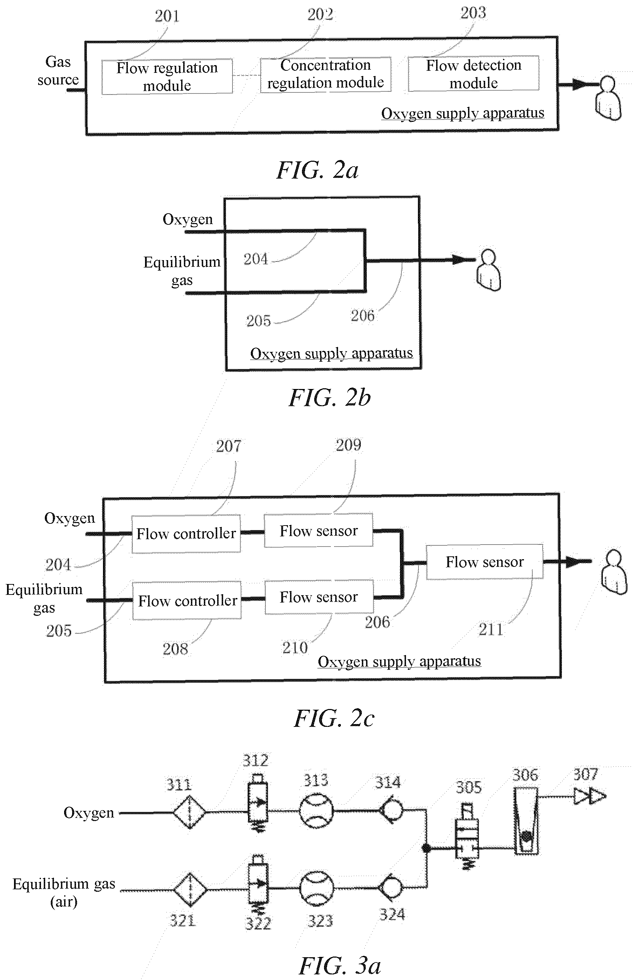

As shown in FIG. 2a, in an embodiment, the oxygen supply apparatus may include a flow regulation module 201 may be used for regulating the flow rate of oxygen-containing gas, a concentration regulation module 202 may be used for regulating the oxygen concentration in the oxygen-containing gas, and a flow detection module 203 may be used for detecting the flow rate of the oxygen-containing gas.

In some embodiments, when the oxygen supply apparatus may provide only one gas path, that is, when the oxygen supply apparatus can only provide oxygen as oxygen-containing gas to a patient, the flow rate of oxygen can be regulated by means of the flow regulation module 201, and the flow rate of oxygen in an actual gas path may be detected by means of the flow detection module 203. At this time, the concentration of oxygen provided by the oxygen gas source may determine the concentration of oxygen in the oxygen-containing gas.

As shown in FIG. 2b, in some embodiments, the oxygen supply apparatus may include an oxygen branch 204, an equilibrium gas branch 205 and a mixed gas path 206. A gas inlet end of the oxygen branch 204 may be in communication with an oxygen input interface, and oxygen may be input from the oxygen input interface. A gas inlet end of the equilibrium gas branch 205 may be in communication with an equilibrium gas input interface, and equilibrium gas (such as air) may be input from the equilibrium gas input interface. Ends of the oxygen branch 204 and the equilibrium gas branch 205 may be in communication with the mixed gas path 206, and the oxygen input through the oxygen branch 204 and the equilibrium gas input through the equilibrium gas branch 205 may be mixed and output through the mixed gas path 206.

In these embodiments, the flow regulation module 201 can respectively regulate the flow rate of oxygen in the oxygen branch 204 and the flow rate of equilibrium gas in the equilibrium gas branch 205; and the flow detection module 203 may detect, in an actual gas path, at least the flow rate of oxygen in the oxygen branch 204 and the flow rate of equilibrium gas in the equilibrium gas branch 205. Of course, if necessary, the flow detection module 203 can also detect the flow rate of oxygen-containing gas in the mixed gas path 206. In general, the concentration of oxygen in the oxygen-containing gas provided by the oxygen supply apparatus can be achieved by respectively regulating the flow rate of oxygen and the flow rate of equilibrium gas. Therefore, during the actual implementation by the concentration regulation module 202 in the solution, the flow regulation module 201 may be used to regulate the flow rate of oxygen and the flow rate of equilibrium gas so as to achieve the purpose of regulating the oxygen concentration.

As shown in FIG. 2c, in an embodiment, the flow regulation module may include a flow controller 207 may be arranged on the oxygen branch 204 to control the flow rate of oxygen, and a flow controller 208 may be arranged on the equilibrium gas branch 205 to control the flow rate of equilibrium gas. The flow detection module may include a flow sensor 209 arranged on the oxygen branch 204 to measure the flow rate of oxygen, a flow sensor 210 may be arranged on the equilibrium gas branch 205 to measure the flow rate of equilibrium gas, and a flow sensor 211 may be arranged on the mixed gas path 206 to measure the flow rate of oxygen-containing gas. Of course, the oxygen supply apparatus may only include the flow sensor 209 may be arranged on the oxygen 204 branch and the flow sensor 210 may be arranged on the equilibrium gas branch 205; or the oxygen supply apparatus may only include the flow sensor 211 may be arrange on the mixed gas path 206.

As shown in FIG. 3a, in an embodiment, a specific structural solution may be used for implementing the oxygen supply apparatus may be provided. A filter 311 may be used for filtering input oxygen, a proportional valve 312 may be used for regulating the flow rate of oxygen, a flow sensor 313 for monitoring the flow rate of oxygen, and a one-way valve 314 may be used for controlling the unidirectional flowing of oxygen may be arranged, in communication in sequence, on the oxygen branch. A filter 321 may be used for filtering input equilibrium gas, a proportional valve 322 may be used for regulating the flow rate of the equilibrium gas, a flow sensor 323 may be used for monitoring the flow rate of the equilibrium gas, and a one-way valve 324 may be used for controlling the unidirectional flowing of the equilibrium gas may be arranged, in communication in sequence, on the equilibrium gas branch. In order to improve the safety, a switch valve 305 may be used for controlling the on-off of the mixed gas path and a glass tube flow meter may be used for monitoring the flow rate of the oxygen-containing gas in the mixed gas path may be further provided on the mixed gas path. When the proportional valve 312/322 fails, the control can be performed by means of the switch valve 305.

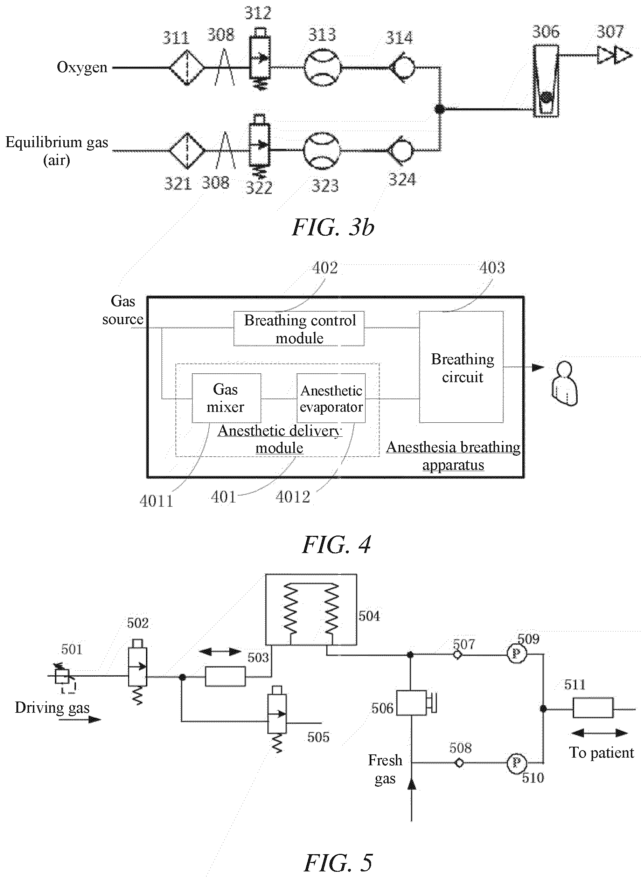

Referring to FIG. 3b, in another embodiment, a mechanical standby valve 308 may be respectively additionally provided in the oxygen branch and in the equilibrium gas branch, such that when the proportional valve 312/322 fails, the control can be performed by means of the mechanical standby valves 308. Of course, a mechanical standby valve can be provided in the mixed gas path, instead of respectively additionally providing a mechanical standby valve 308 in the oxygen branch and in the equilibrium gas branch, such that when the proportional valve 312/322 fails, the mixed gas path can be disconnected by means of the mechanical standby valve.

It should be noted that in other embodiments, the devices provided in FIG. 3 can be replaced by other devices with the same functions, for example, the replacement between a mechanical device and an electronic device. In the embodiments mentioned above, the function of the flow controller can be implemented by either an electronic device or a mechanical device.

As shown in FIG. 4, the anesthesia breathing apparatus may include an anesthetic delivery module 401, a breathing control module 402 and a breathing circuit 403. the anesthetic delivery module 401 may be connected to the gas source interface and the breathing circuit 403, and may deliver an anesthetic to the breathing circuit 403; and the breathing control module 402 may be connected to the gas source interface and the breathing circuit 403, and may provide breathing support to the patient through the breathing circuit 403.

The anesthetic delivery module 401 may include a gas mixer 4011 and an anesthetic evaporator 4012. The gas mixer 4011 may be connected to the gas source interface and the anesthetic evaporator 4012, and the oxygen and the equilibrium gas may be mixed and then output to the anesthetic evaporator 4012. By means of the anesthetic evaporator 4012, the anesthetic may be mixed with the gas output by the gas mixer 4011 and then may be output to the breathing circuit 403.

As shown in FIG. 5, in an embodiment, a specific structural solution may be used for implementing the breathing control module 402 and the breathing circuit 403 may be provided. One end of a pressure reducing valve 501 may be connected to the gas source interface to input a driving gas and may reduce pressure of the driving gas. An inhalation valve 502 may supply air to a bellows 504 after being switched on. A flow sensor 503 may be configured to detect the flow rate of the current gas path. The driving gas may enter an outer cavity of the bellows 504 via the pressure reducing valve 501, the inhalation valve 502 and the flow sensor 503 and may compress a gasbag in the bellows 504 to move downwards, and the gas in the gasbag may flow into the patient's lungs through the breathing circuit. At this time, the pressure in the breathing circuit should gradually increase with the gas supply. However, if the pressure may increase to exceed the shut-off pressure of an exhalation valve 505, even if the inhalation valve 502 may continue to supply gas, the excess driving gas will be discharged from the exhalation valve 505, thereby ensuring that the airway pressure at the patient end will not exceed a set control pressure. During exhalation, the shut-off pressure of the exhalation valve 505 may be released, the pressure in the circuit will drop rapidly, and the folded gasbag in the bellows 504 will rise to the top, waiting for a next breathing cycle. One-way valves 507 and 508 may be provided in the breathing circuit. Pressure sensors 509 and 510 may be provided in the breathing circuit. A flow sensor 511 may be provided to measure the flow rate of breathing gas of the patient. A carbon dioxide absorption tank 506 may be provided to absorb carbon dioxide in the exhaled gas of the patient. **In** FIG. 5, a fresh gas input end may be an output end of the anesthetic delivery module for delivering fresh gas (carrying an anesthetic) to the patient.

Specifically, some conventional solutions in the prior art can be used as the specific structural implementation solutions of the anesthesia breathing apparatus. For example, the solution in the Chinese patent application No. CN 200710075839.1, entitled " 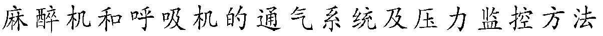 [VENTILATION SYSTEM AND PRESSURE MONITORING METHOD OF ANESTHESIA MACHINE AND VENTILATOR]", is used; or the solution in the Chinese patent application No. CN 201480017240.0, entitled " 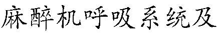 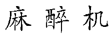 [RESPIRATORY SYSTEM OF ANESTHESIA MACHINE AND ANESTHESIA MACHINE]", may be used; or pneumatic pneumatically-controlled, pneumatic electrically-controlled or electric electrically-controlled mode may be used; or a closed system, a semi-closed system, a semi-open system, an open system, etc. may be used for a circuit mode.

For the anesthesia machine, the flow rate of the breathing gas provided to the patient may be limited, and should be set in consideration of the patient's physiological conditions. In general, the flow rate of breathing gas selected by the anesthesia machine may be set according to the patient's body weight. Therefore, the flow rate may be generally small, and even 300 milliliters per minute provided can basically meet the needs of oxygen metabolism of the patient. It may be generally considered that more than 4 liters per minute may be an extremely high flow rate.

During an oxygen supply operation, in order to extend the asphyxia time window as far as possible to reserve enough time for intubation by a surgeon, an anesthesiologist will use a manual mode of the anesthesia machine to generally regulate the oxygen flow rate to 4-8 L/min, and then after oxygen supply to the patient for a period of time in the manual mode, the intubation for the patient may be performed. For a normal adult, there may be an asphyxia time window of about 8 minutes available for intubation. However, for obese patients and obstetrical patients having a high incidence of difficult airway. after sufficient oxygen supply, after administration with a muscle relaxant, the oxygen saturation (SPO2) may drop to 80% in only 1.5-4 minutes, such that the intubation risk may be high, and the airway must be established within a short asphyxia time window. It may be very dangerous for emergency intubation for difficult airway, and if many intubation attempts may be unsuccessful, the patient may not be able to undergo intubation or ventilation, endangering the patient's life. The surgeon will be under great stress.

At present, in some operations, a jet ventilator may be used for ventilation for the patient. For example, during the respiratory dilatation for a patient with stenosis of the lower respiratory tract (SGS), the surgeon and the anesthetist should share the airway management, which may be a great challenge for them. At present, there may be no fixed standard for ventilation during surgery, and ventilation with a jet ventilator may be widely used. However, high-frequency jet gas supply through glottis may cause risks of emphysema, pneumothorax, and hypoxia of the patient.

In order to solve the above problem, in the present application, not only an oxygen supply apparatus may be integrated in the anesthesia machine, but also the solution that the oxygen supply apparatus may provide oxygen-containing gas to a patient at a flow rate greater than 15 liters per minute (also referred as high-flow oxygen supply in the present application) may be further creatively proposed.

It should be noted that the 15 liters per minute in this embodiment may refer to the flow rate of the oxygen-containing gas provided to the patient. When the oxygen supply apparatus only uses oxygen, it may mean that the flow rate of oxygen may be not less than 15 liters per minute; and when the oxygen supply apparatus uses a mixed gas of oxygen and air as the oxygen-containing gas, it may mean that the total flow rate of the mixed gas may be not less than 15 liters per minute.

Ventilation of the patient at a flow rate greater than 15 liters per minute can significantly extend the asphyxia time window, which has extremely high clinical value. For example, for a patient with difficult airway, the preoperative pre-oxygenation and high-flow oxygen supply during intubation (the flow rate reaches 40-70 L/min) can significantly extend the asphyxia time window, and the average asphyxia time reaches 17 min, such that the surgeon can easily establish artificial airway. Moreover, for some patients undergoing laryngeal surgery in a short time (within half an hour), the whole operation can be completed even by directly using high-flow oxygen supply, thereby reducing the intubation step, thereby reducing the workload of a surgeon, and releasing the patient from pain due to intubation.

**In** some embodiments, the oxygen supply apparatus of the anesthesia machine may be only configured to provide oxygen, that is, when there is only one gas path for delivering oxygen, the high flow rate may be defined as long as the flow rate may be not less than 15 liters per minute. **In** other embodiments, when the oxygen supply apparatus of the anesthesia machine includes an oxygen branch and an equilibrium gas branch, the high flow rate may be defined as the total flow rate of the mixed oxygen and equilibrium gas being greater than 30 liters per minute, and at this time, the flow rate of oxygen may not be limited to be greater than 15 liters per minute.

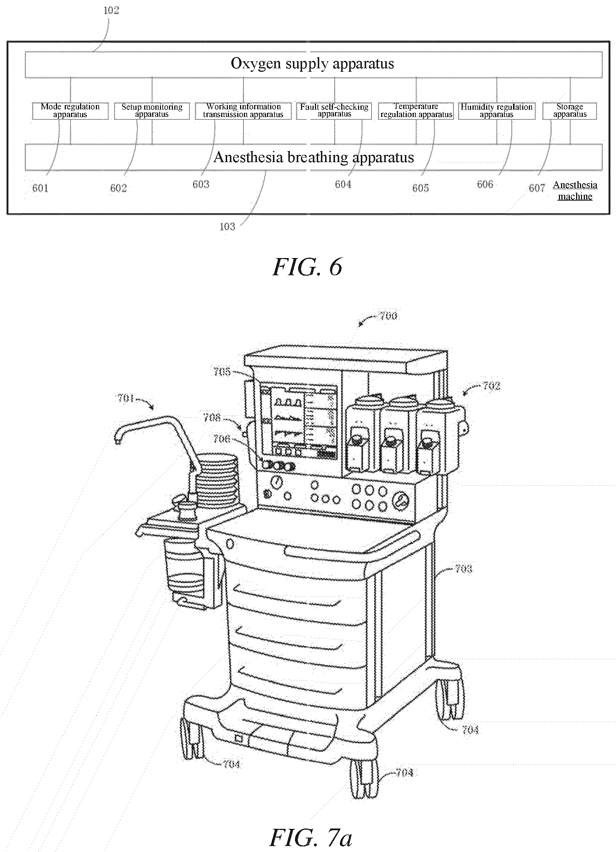

As shown in FIG. 6 (only showing the control and connection relationship between the apparatuses of the anesthesia machine), in an embodiment, the anesthesia machine may further include a mode regulation apparatus 601, a setup monitoring apparatus 602, a working information transmission apparatus 603, a fault self-checking apparatus 604, a temperature regulation apparatus 605, a humidity regulation apparatus 606, and a storage apparatus 607.

The mode regulation apparatus 601 may respond to a received operation instruction to control the oxygen supply apparatus to switch between at least two working modes.

**In** an embodiment, the working modes may include a low-flow mode and a high-flow mode; the low-flow mode may be defined as the oxygen supply apparatus providing oxygen-containing gas to a patient at a flow rate less than 15 liters per minute, and the high-flow mode may be defined as the oxygen supply apparatus providing oxygen-containing gas to a patient at a flow rate greater than 15 liters per minute.

Further, the working modes may also include a child mode and an adult mode. The child mode may be defined as that the oxygen supply apparatus providing oxygen-containing gas to a patient at a flow rate less than 20 liters per minute, and the adult mode may be defined as the oxygen supply apparatus providing oxygen-containing gas to the patient at a flow rate of 1-100 liters per minute.

The oxygen supply apparatus may provide, to a patient, oxygen-containing gas at different flow rate and/or concentration in different working modes. Specifically, different working modes can be set according to different requirements, for example, introducing high-flow oxygen-containing gas before anesthesia and introducing low-flow oxygen-containing gas after surgery; or different working modes can be set for different types of patient, for example, setting a larger flow rate for an adult, and a small flow rate for a child. The oxygen concentration may be usually in the range of 21% to 100%.

Through the setting of the working modes, a user can make a quick setup according to different needs.

The setup monitoring apparatus 602 generates alarm prompt information when monitoring that a flow rate and/or an oxygen concentration setup value of the oxygen-containing gas input by a user may be not within a range corresponding to the current working mode. For example, when the current working mode is the child mode, the oxygen supply apparatus may provide the oxygen-containing gas to the patient at a flow rate less than 20 liters per minute. At this time, if the user manually set the flow rate to more than 20 liters per minute due to inattention or wrong operation, or the actual flow rate of the oxygen-containing gas may be greater than 20 liters per minute due to the device failure of the anesthesia machine, the setup monitoring apparatus 602 will acquire the information and give an alarm prompt to avoid accidents.

The working information transmission apparatus 603 may acquire working information of the oxygen supply apparatus and/or the anesthesia breathing apparatus, and send the working information. The working information may include gas path flow information, oxygen concentration information, pressure information, device work duration information, working mode information, fault information, etc. The obtained working information can be sent, by the working information transmission apparatus 603, to a monitor of the anesthesia machine for display, or to other monitoring devices, or to an information system for use by relevant personnel. **In** the anesthesia machine provided by the present application, the working information of the oxygen supply apparatus and the anesthesia breathing apparatus can be shared and integrated. For example, the information such as the oxygen supply mode and setup information can be directly reflected on a routine information record sheet of the anesthesia machine, thereby reducing the workload of manual recording by the surgeon.

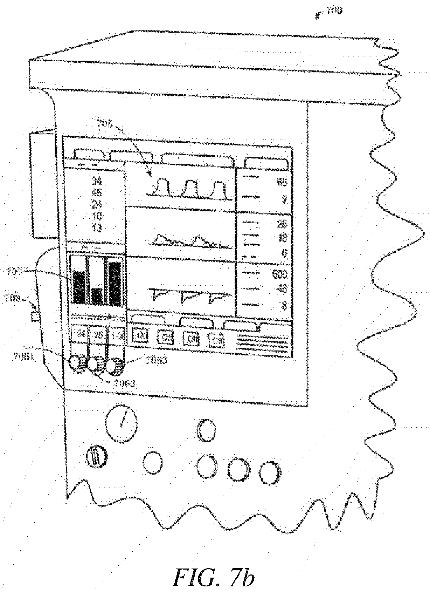

The fault self-checking apparatus 604 may monitor fault information of the oxygen supply apparatus and/or the anesthesia breathing apparatus. Specifically, the fault self-checking apparatus 604 can also be configured to give an alarm prompt after detecting a fault. The fault information may include gas path blockage, flow regulation failure, concentration regulation failure and other information. Specifically, the fault information can also be displayed on an electronic display (as shown in FIGS. 7a and 7b) of the anesthesia machine to facilitate fault location.

The temperature regulation apparatus 605 may regulate a temperature of the oxygen-containing gas output by the oxygen supply apparatus, and the humidity regulation apparatus 606 may regulate a humidity of the oxygen-containing gas output by the oxygen supply apparatus, so as to provide warm and humid gas flow to the patient and reduce the irritation to the patient. Of course, in another embodiment, the temperature regulation apparatus 605 and the humidity regulation apparatus 606 can also be configured to regulate the temperature and the humidity of the breathing gas provided to the patient by the anesthesia breathing apparatus. Of course, the temperature regulation apparatus 605 and the humidity regulation apparatus 606 can also be integrated together. Specifically, the humidity regulation apparatus may be integrated inside the anesthesia machine, and may also be implemented by a humidifier externally mounted on the anesthesia machine. Gas outlet ends of the oxygen supply apparatus and the anesthesia breathing apparatus may be connected to an input end of the humidifier, and the humidifier may humidify the gas and then outputs the gas to the patient.

The storage apparatus 607 may store the working information of the oxygen supply apparatus and/or the anesthesia breathing apparatus.

**In** the embodiment of the present application, since the oxygen supply apparatus and the anesthesia breathing apparatus may be independent and can work independently, the mode regulation apparatus 601, the setup monitoring apparatus 602, the working information transmission apparatus 603, the fault self-checking apparatus 604, the temperature regulation apparatus 605, the humidity regulation apparatus 606 and the storage apparatus 607 can be configured to work when the oxygen supply apparatus and the anesthesia breathing apparatus simultaneously operate or when one of them operates.

FIG. 7a is a schematic perspective diagram of an anesthesia machine. The anesthesia machine 700 may be provided with three flow regulation selectors 706 configured to respectively control flow rates of oxygen, laughing gas and air. The anesthesia machine 700 may include an anesthesia breathing apparatus 701, an anesthetic evaporator 702 and other anesthesia transmission system elements. The anesthesia machine 700 can be loaded on a cart 703, which can be provided with wheels 704 to facilitate movement. An electronic display 705 can be configured to visually provide, to a user, information about the flow rate of gas or the anesthesia delivery procedure and state. The electronic display 705 may also be a display with a touch function, and the user can touch the electronic display 705 to control some of the functions of the anesthesia machine, such as regulating the flow rate of gas and the oxygen concentration. An ACGO outlet 708 of the anesthesia machine may be provided.

FIG. 7b is a schematic partial enlarged diagram of the anesthesia machine shown in FIG. 7a. Electronic flow-selection knobs 7061, 7062 and 7063 of the anesthesia machine 700 can be configured as mechanical flow-selection knobs in the manual regulation mode, that is, the flow rate of gas can be regulated by the knobs 7061, 7062 and 7063 in any mode. As shown in the figure, monitored patient parameters and other related information can be presented to the surgeon through the display 705. The display 705 may also be a display with a touch screen, and the surgeon can control the operation of the anesthesia machine 700 through the display 705. In a specific embodiment, the flow-selection knob 7061 can be configured to control the flow rate of oxygen, the flow-selection knob 7062 can be configured to control the flow rate of laughing gas, and the flow-selection knob 7063 can be configured to control the flow rate of air, such that the user can rotate the knobs to achieve the desired flow rate of gas.

A flow display unit 707 can be configured to visually display the current flow rate of the regulated gas. The flow display unit 707 may be of an electronic type, such as a flow tube simulated on a display screen, or may be a mechanical flow tube; or two types of flow display units may be simultaneously provided in the system; or the flow display unit 707 may be switched to a corresponding display mode in different operation modes. For example, the flow display unit displays the flow rate in an electronic display manner when in an electric control mode, and displays the flow rate of the regulated gas in a mechanical scale may change when in a manual control mode, such that the flow rate of gas can be normally displayed in both the electric control mode and the manual control mode. As shown in the figure, each of the flow-selection knobs 7061, 7062 and 7063 may respectively correspond to a flow tube, such that the flow rate of each gas can be displayed independently.

Although the anesthesia machine with three flow-selection knobs is shown in the figure, it can be understood that more or less knobs can be provided as desired, which does not mean that this disclosure is only limited to providing three flow-selection knobs. For example, two groups of flow-selection knobs may be provided to respectively regulate the flow rates of gas in the oxygen supply apparatus and in the anesthesia breathing apparatus in the above embodiments. The regulated objects may be not limited to the three kinds of gas, and can be more or less, and the fluids other than gas may also be controlled. Although the figure shows that one knob correspondingly regulates the flow rate of one kind of gas, in some embodiments, the flow rate of certain gas can be regulated with more than one knob, or with a multiplexing/switching design of the internal mechanical structure, one knob can be configured to regulate the flow rates of multiple kinds of gas. For example, when a knob for regulating the oxygen concentration is used, the oxygen concentration may be regulated by respectively regulating the flow rates of oxygen and equilibrium gas. Of course, the function of the flow-selection knob can also be implemented on the electronic display 705 with a touch function.

An embodiment of the present application further may provide an anesthesia machine system, may include an anesthesia machine and an oxygen supply device, the anesthesia machine may be provided with a fixing mechanism, and the oxygen supply device being detachably fixed to the anesthesia machine via the fixing mechanism. The oxygen supply device may include a housing and the above oxygen supply apparatus may be arranged in the housing, and the gas path structure thereof can refer to the oxygen supply apparatus provided in the above embodiments, which will not be described in detail here. The fixing mechanism can be a fixing platform, a fixing hook, or a structure conventionally used in the art for detachable fixing between two devices.

**In** some embodiments, the anesthesia machine and the oxygen supply device can be powered by two independent power sources. **In** other embodiments, a power source input interface of the oxygen supply device may be connected to an auxiliary output power source interface of the anesthesia machine, and the oxygen supply device may be powered through the auxiliary output power source interface of the anesthesia machine.

**In** some embodiments, a gas source input interface of the oxygen supply device may be connected to an external gas source, that is, the oxygen supply device and the anesthesia machine may respectively have independent external gas sources; or the gas source input interface of the oxygen supply device may be connected to a gas source of the anesthesia machine.

Further, the anesthesia machine can communicate with the oxygen supply device in a wired or wireless way to implement information sharing therebetween. For example, the fixing mechanism may be further provided with a first information transmission interface and a second information transmission interface, the oxygen supply device may be provided with a third information transmission interface at a corresponding position, and the anesthesia machine may be provided with a fourth information transmission interface at a corresponding position. The first information transmission interface on the fixing mechanism may be connected to the fourth information transmission interface on the anesthesia machine, and when the oxygen supply device may be installed on the fixing mechanism, the third information transmission interface on the oxygen supply device may be connected to the second information transmission interface on the fixing structure, thereby implementing data transmission between the oxygen supply device and the anesthesia machine. The working information of the anesthesia machine and the oxygen supply device can also be displayed on a display located on one of them, or a user can control the anesthesia machine and the oxygen supply device through a touch display on one of the anesthesia machine and the oxygen supply device.

It should be noted that the mode regulation apparatus 601, the setup monitoring apparatus 602, the working information transmission apparatus 603, the fault self-checking apparatus 604, the temperature regulation apparatus 605, the humidity regulation apparatus 606 and the storage apparatus 607 of the anesthesia machine provided by the above embodiments can also be applied to the anesthesia machine system provided in this embodiment. For example, the mode regulation apparatus 601, the setup monitoring apparatus 602, the working information transmission apparatus 603, the fault self-checking apparatus 604, the temperature regulation apparatus 605, the humidity regulation apparatus 606, and the storage apparatus 607 may be arranged on the oxygen supply device and/or the anesthesia machine to implement the same function.

In addition, in order to implement high-flow oxygen supply by the anesthesia machine system provided in this embodiment, the oxygen supply device can be configured to provide oxygen-containing gas to a patient at a flow rate greater than 15 liters per minute.

The anesthesia machine system provided in this embodiment can conveniently use the anesthesia machine to perform anesthesia breathing support to the patient, and use the oxygen supply device to supply oxygen to the patient.

The above-mentioned examples merely represent several embodiments, giving specifics and details thereof, but should not be understood as limiting the scope of the present disclosure thereby. It should be noted that those of ordinary skill in the art would have also made several variations and improvements without departing from the concept of the present disclosure, and these variations and improvements would all fall within the scope of protection of the present disclosure. Therefore, the scope of protection of the present disclosure shall be in accordance with the appended claims.

## Claims

1. An anesthesia machine (100), comprising a gas source interface (101), and an anesthesia breathing apparatus (103) which is connected to the gas source interface (101), wherein
the anesthesia breathing apparatus (103) comprises an anesthetic delivery module (401), a breathing control module (402) and a breathing circuit (403);
the anesthetic delivery module (401) is connected to the gas source interface (101) and the breathing circuit (403), and delivers an anesthetic into the breathing circuit (403); and
the breathing control module (402) is connected to the gas source interface (101) and the breathing circuit (403), and provides breathing support to the patient through the breathing circuit (403);
the anesthesia machine (100) further comprising an oxygen supply apparatus (102) which is integrated inside the anesthesia machine and connected to the gas source interface (101), wherein
the oxygen supply apparatus (102) and the anesthesia breathing apparatus (103) respectively have independent gas outlets;
the oxygen supply apparatus (102) provides an oxygen-containing gas to a patient, the oxygen-containing gas is an oxygen or a mixed gas of an oxygen and an equilibrium gas; and the oxygen supply apparatus (102) comprises a flow regulation module (201) for regulating the flow rate of the oxygen-containing gas, and
when the oxygen-containing gas is an oxygen, the flow regulation module (201) is configured to control the oxygen-containing gas with a flow rate greater than or equal to 30 liters per minute; or
when the oxygen-containing gas is a mixed gas of an oxygen and an equilibrium gas, the flow regulation module (201) is configured to control the oxygen-containing gas with a flow rate greater than or equal to 30 liters per minute; and the gas source interface (101) further comprises an oxygen input interface (101a) for providing oxygen and an equilibrium gas input interface (101b) for providing equilibrium gas; the oxygen supply apparatus (102) further comprises an oxygen branch (204), an equilibrium gas branch (205) and a mixed gas path (206); a gas inlet end of the oxygen branch (204) is in communication with the oxygen input interface (101a), and oxygen is inputted from the oxygen input interface (101a); a gas inlet end of the equilibrium gas branch (205) is in communication with the equilibrium gas input interface (101b), and equilibrium gas is inputted from the equilibrium gas input interface (101b); and outlet ends of the oxygen branch (204) and the equilibrium gas branch (205) are in communication with the mixed gas path (206), and the oxygen input from the oxygen branch (204) and the equilibrium gas input from the equilibrium gas branch (205) are mixed and outputted through the mixed gas path (206).

2. The anesthesia machine (100) of claim 1, further comprising a mode regulation apparatus (601), which responds to a received operation instruction to control the oxygen supply apparatus (102) to switch between at least two working modes.

3. The anesthesia machine (100) of claim 2, further comprising a setup monitoring apparatus (602), which generates alarm prompt information when a setup value of flow rate and/or oxygen concentration of the oxygen-containing gas inputted by a user is monitored not to be within a range corresponding to a current working mode.

4. The anesthesia machine (100) of claim 1, further comprising a working information transmission apparatus (603), which acquires working information of the oxygen supply apparatus (102) and/or the anesthesia breathing apparatus (103) and transmits the working information.

5. The anesthesia machine (100) of claim 1, further comprising a fault self-checking apparatus (604), which monitors fault information of the oxygen supply apparatus (102) and/or the anesthesia breathing apparatus (103).

6. The anesthesia machine (100) of claim 1, further comprising a temperature regulation apparatus (605) and/or a humidity regulation apparatus (606), wherein the temperature regulation apparatus (605) regulates a temperature of the oxygen-containing gas outputted by the oxygen supply apparatus (102), and/or the humidity regulation apparatus (606) regulates a humidity of the oxygen-containing gas outputted by the oxygen supply apparatus (102).

7. The anesthesia machine (100) of any one of claim 1-6, wherein the oxygen supply apparatus (102) comprises a concentration regulation module (202) for regulating an oxygen concentration in the oxygen-containing gas, and/or a flow detection module (203) for detecting the flow rate of the oxygen-containing gas.

8. The anesthesia machine (100) of claim 1, wherein the anesthetic delivery module (401) comprises a gas mixer (4011) and an anesthetic evaporator (4012); the gas mixer (4011) is connected to the gas source interface (101) and the anesthetic evaporator (4012), and the oxygen and the equilibrium gas are mixed and then outputted to the anesthetic evaporator (4012); and the anesthetic is mixed with the gas provided by the gas mixer (4011) and then outputted to the breathing circuit (403) by the anesthetic evaporator (4012).

9. An anesthesia machine system, comprising an anesthesia machine and an oxygen supply device, wherein
the anesthesia machine is provided with a fixing mechanism, and the oxygen supply device is detachably fixed to the anesthesia machine by means of the fixing mechanism; wherein the oxygen supply device provides an oxygen-containing gas to a patient, the oxygen-containing gas is an oxygen or a mixed gas of an oxygen and an equilibrium gas; and
when the oxygen-containing gas is an oxygen, the oxygen supply device is configured to control the oxygen-containing gas with a flow rate greater than or equal to 30 liters per minute; or
when the oxygen-containing gas is a mixed gas of an oxygen and an equilibrium gas, the oxygen supply device is configured to control the oxygen-containing gas with a flow rate greater than or equal to 30 liters per minute; and the oxygen supply device further comprises an oxygen branch (204), an equilibrium gas branch (205) and a mixed gas path (206); a gas inlet end of the oxygen branch (204) is in communication with an oxygen input interface (101a), and oxygen is inputted from the oxygen input interface (101a); a gas inlet end of the equilibrium gas branch (205) is in communication with an equilibrium gas input interface (101b), and equilibrium gas is inputted from the equilibrium gas input interface (101b); and outlet ends of the oxygen branch (204) and the equilibrium gas branch (205) are in communication with the mixed gas path (206), and the oxygen input from the oxygen branch (204) and the equilibrium gas input from the equilibrium gas branch (205) are mixed and outputted through the mixed gas path (206);
the anesthesia machine is in communication with the oxygen supply device in a wired or wireless manner;
the working information of the anesthesia machine and the oxygen supply device is displayed on a display located on one of them, or a user is able to control the anesthesia machine and the oxygen supply device through a touch display on one of them.

10. The anesthesia machine system of claim 9, wherein a power source input interface of the oxygen supply device is connected to an auxiliary output power source interface of the anesthesia machine.

11. The anesthesia machine system of claim 9, wherein a gas source input interface of the oxygen supply device is connected to an external gas source or connected to a gas source of the anesthesia machine.

## Patentansprüche

1. Ein Anästhesiegerät (100), das eine Gasquellenschnittstelle (101) und ein Anästhesieatemgerät (103) umfasst, das mit der Gasquellenschnittstelle (101) verbunden ist, wobei
das Anästhesieatemgerät (103) ein Anästhetikumabgabemodul (401), ein Atemsteuerungsmodul (402) und einen Atemkreislauf (403) umfasst;
das Anästhetikumabgabemodul (401) mit der Gasquellenschnittstelle (101) und dem Atemkreislauf (403) verbunden ist und ein Anästhetikum in den Atemkreislauf (403) abgibt; und
das Atemsteuerungsmodul (402) mit der Gasquellenschnittstelle (101) und dem Atemkreislauf (403) verbunden ist und dem Patienten über den Atemkreislauf (403) Atemunterstützung leistet;
das Anästhesiegerät (100) ferner mit einer Sauerstoffzufuhrvorrichtung (102) ausgestattet ist, die in das Anästhesiegerät integriert und mit der Gasquellenschnittstelle (101) verbunden ist, wobei
die Sauerstoffzufuhrvorrichtung (102) und das Anästhesieatemgerät (103) jeweils über unabhängige Gasauslässe verfügen;
die Sauerstoffzufuhrvorrichtung (102) einem Patienten ein sauerstoffhaltiges Gas zuführt, wobei das sauerstoffhaltige Gas Sauerstoff oder ein Gasgemisch aus Sauerstoff und einem Gleichgewichtsgas ist; und die Sauerstoffzufuhrvorrichtung (102) ein Durchflussregelmodul (201) zur Regelung der Durchflussrate des sauerstoffhaltigen Gases umfasst, und
wenn es sich bei dem sauerstoffhaltigen Gas um Sauerstoff handelt, das Durchflussregelmodul (201) so ausgelegt ist, dass es das sauerstoffhaltige Gas mit einer Durchflussrate von mindestens 30 Litern pro Minute regelt; oder
wenn das sauerstoffhaltige Gas ein Gasgemisch aus Sauerstoff und einem Gleichgewichtsgas ist, das Durchflussregelmodul (201) so ausgelegt ist, dass es das sauerstoffhaltige Gas mit einer Durchflussrate von mindestens 30 Litern pro Minute regelt; und die Gasquellenschnittstelle (101) ferner eine Sauerstoff-Eingangsschnittstelle (101a) zur Bereitstellung von Sauerstoff und eine Gleichgewichtsgas-Eingangsschnittstelle (101b) zur Bereitstellung von Gleichgewichtsgas umfasst;
die Sauerstoffzufuhrvorrichtung (102) ferner einen Sauerstoffzweig (204) umfasst, einen Gleichgewichtsgaszweig (205) und einen Mischgasweg (206); ein Gaseinlassende des Sauerstoffzweigs (204) mit der Sauerstoff-Eingangsschnittstelle (101a) in Verbindung steht, und Sauerstoff von der Sauerstoff-Eingangsschnittstelle (101a) zugeführt wird; ein Gaseinlassende des Gleichgewichtsgaszweigs (205) mit der Gleichgewichtsgas-Eingangsschnittstelle (101b) in Verbindung steht, und Gleichgewichtsgas von der Gleichgewichtsgas-Eingangsschnittstelle (101b) zugeführt wird; und die Auslassenden des Sauerstoffzweigs (204) und des Gleichgewichtsgaszweigs (205) mit dem Mischgasweg (206) in Verbindung stehen, und der Sauerstoff, der aus dem Sauerstoffzweig (204) zugeführt wird, sowie das Gleichgewichtsgas, das aus dem Gleichgewichtsgaszweig (205) zugeführt wird, gemischt und über den Mischgasweg (206) abgegeben werden.

2. Das Anästhesiegerät (100) nach Anspruch 1, das ferner eine Modusregelungsvorrichtung (601) umfasst, die auf einen empfangenen Betriebsbefehl reagiert, um die Sauerstoffzufuhrvorrichtung (102) so zu steuern, dass sie zwischen mindestens zwei Betriebsmodi umschaltet.

3. Das Anästhesiegerät (100) nach Anspruch 2, das ferner eine Einstellungsüberwachungsvorrichtung (602) umfasst, die Alarmhinweise generiert, wenn ein von einem Benutzer eingegebener Einstellwert für die Durchflussrate und/oder die Sauerstoffkonzentration des sauerstoffhaltigen Gases überwacht wird und sich nicht innerhalb eines Bereichs befindet, der einem aktuellen Betriebsmodus entspricht.

4. Das Anästhesiegerät (100) nach Anspruch 1, das ferner eine Betriebsinformationsübertragungsvorrichtung (603) umfasst, die Betriebsinformationen der Sauerstoffzufuhrvorrichtung (102) und/oder des Anästhesieatemgeräts (103) erfasst und die Betriebsinformationen überträgt.

5. Das Anästhesiegerät (100) nach Anspruch 1, das ferner eine Fehler-Selbstprüfvorrichtung (604) umfasst, die Fehlerinformationen der Sauerstoffzufuhrvorrichtung (102) und/oder des Anästhesieatemgeräts (103) überwacht.

6. Das Anästhesiegerät (100) nach Anspruch 1, das ferner eine Temperaturregelungsvorrichtung (605) und/oder eine Feuchtigkeitsregelungsvorrichtung (606) umfasst, wobei die Temperaturregelungsvorrichtung (605) eine Temperatur des von der Sauerstoffzufuhrvorrichtung (102) abgegebenen sauerstoffhaltigen Gases regelt, und/oder die Feuchtigkeitsregelungsvorrichtung (606) eine Feuchtigkeit des von der Sauerstoffzufuhrvorrichtung (102) abgegebenen sauerstoffhaltigen Gases regelt.

7. Das Anästhesiegerät (100) nach einem der Ansprüche 1 bis 6, wobei die Sauerstoffzufuhrvorrichtung (102) ein Konzentrationsregelungsmodul (202) zum Regeln einer Sauerstoffkonzentration in dem sauerstoffhaltigen Gas und/oder ein Durchflusserfassungsmodul (203) zum Erfassen der Durchflussrate des sauerstoffhaltigen Gases umfasst.

8. Das Anästhesiegerät (100) nach Anspruch 1, wobei das Anästhetikumabgabemodul (401) einen Gasmischer (4011) und einen Anästhetikumverdampfer (4012) umfasst; der Gasmischer (4011) mit der Gasquellenschnittstelle (101) und dem Anästhetikumverdampfer (4012) verbunden ist, und der Sauerstoff und das Gleichgewichtsgas gemischt und anschließend an den Anästhetikumverdampfer (4012) abgegeben werden; und das Anästhetikum mit dem vom Gasmischer (4011) bereitgestellten Gas gemischt und anschließend vom Anästhetikumverdampfer (4012) an den Atemkreislauf (403) abgegeben wird.

9. Ein Anästhesiegerätesystem, das ein Anästhesiegerät und eine Sauerstoffzufuhrvorrichtung umfasst, wobei das Anästhesiegerät mit einem Befestigungsmechanismus versehen ist und die Sauerstoffzufuhrvorrichtung mittels des Befestigungsmechanismus lösbar an dem Anästhesiegerät befestigt ist; wobei die Sauerstoffzufuhrvorrichtung einem Patienten ein sauerstoffhaltiges Gas zuführt, das sauerstoffhaltige Gas Sauerstoff oder ein Gemisch aus Sauerstoff und einem Gleichgewichtsgas ist; und wenn das sauerstoffhaltige Gas Sauerstoff ist, die Sauerstoffzufuhrvorrichtung so ausgelegt ist, dass sie das sauerstoffhaltige Gas mit einer Durchflussrate von mindestens 30 Litern pro Minute bereitstellt; oder
wenn es sich bei dem sauerstoffhaltigen Gas um ein Gemisch aus Sauerstoff und einem Gleichgewichtsgas handelt, die Sauerstoffzufuhrvorrichtung so ausgelegt ist, dass sie das sauerstoffhaltige Gas mit einer Durchflussrate von mindestens 30 Litern pro Minute bereitstellt;
und die Sauerstoffzufuhrvorrichtung ferner einen Sauerstoffzweig (204), einen Gleichgewichtsgaszweig (205) und einen Mischgasweg (206) umfasst; ein Gaseinlassende des Sauerstoffzweigs (204) mit einer Sauerstoff-Eingangsschnittstelle (101a) in Verbindung steht und Sauerstoff von der Sauerstoff-Eingangsschnittstelle (101a) zugeführt wird; ein Gaseinlassende des Gleichgewichtsgaszweigs (205) mit einer Gleichgewichtsgas-Eingangsschnittstelle (101b) in Verbindung steht und Gleichgewichtsgas von der Gleichgewichtsgas-Eingangsschnittstelle (101b) zugeführt wird; und die Auslassenden des Sauerstoffzweigs (204) und des Gleichgewichtsgaszweigs (205) mit dem Mischgasweg (206) in Verbindung stehen, und der Sauerstoff, der aus dem Sauerstoffzweig (204) zugeführt wird, sowie das Gleichgewichtsgas, das aus dem Gleichgewichtsgaszweig (205) zugeführt wird, gemischt und über den Mischgasweg (206) abgegeben werden;
das Anästhesiegerät über eine kabelgebundene oder kabellose Verbindung mit der Sauerstoffzufuhrvorrichtung in Verbindung steht;
die Betriebsdaten des Anästhesiegeräts und der Sauerstoffzufuhrvorrichtung auf einem Display angezeigt werden, das sich an einem der Geräte befindet, oder der Benutzer das Anästhesiegerät und die Sauerstoffzufuhrvorrichtung über ein Touchdisplay an einem der Geräte steuern kann.

10. Das Anästhesiegerätesystem nach Anspruch 9, wobei eine Stromversorgungs-Eingangsschnittstelle der Sauerstoffzufuhrvorrichtung mit einer Hilfsausgangs-Stromversorgungsschnittstelle des Anästhesiegeräts verbunden ist.

11. Das Anästhesiegerätesystem nach Anspruch 9, wobei eine Gasquellen-Eingangsschnittstelle der Sauerstoffzufuhrvorrichtung mit einer externen Gasquelle oder mit einer Gasquelle des Anästhesiegeräts verbunden ist.

## Revendications

1. Machine d'anesthésie (100), comprenant une interface de source de gaz (101), et un appareil de respiration d'anesthésie (103) lequel est relié à l'interface de source de gaz (101), où
l'appareil de respiration d'anesthésie (103) comprend un module de délivrance d'anesthésique (401), un module de contrôle de respiration (402) et un circuit de respiration (403) ;
le module de délivrance d'anesthésique (401) est relié à l'interface de source de gaz (101) et au circuit de respiration (403), et délivre un anesthésique dans le circuit de respiration (403) ; et
le module de contrôle de respiration (402) est relié à l'interface de source de gaz (101) et au circuit de respiration (403), et fournit une assistance respiratoire au patient par le biais du circuit de respiration (403) ;
la machine d'anesthésie (100) comprenant en outre un appareil de fourniture d'oxygène (102) lequel est intégré dans la machine d'anesthésie et est relié à l'interface de source de gaz (101), où
l'appareil de fourniture d'oxygène (102) et l'appareil de respiration d'anesthésie (103) ont respectivement des sorties de gaz indépendantes ;
l'appareil de fourniture d'oxygène (102) fournit un gaz contenant de l'oxygène à un patient, le gaz contenant de l'oxygène est de l'oxygène ou un gaz mixte d'oxygène et d'un gaz d'équilibre ; et l'appareil de fourniture d'oxygène (102) comprend un module de régulation de débit (201) destiné à réguler le débit du gaz contenant de l'oxygène, et
lorsque le gaz contenant de l'oxygène est de l'oxygène, le module de régulation de débit (201) est configuré pour réguler le gaz contenant de l'oxygène à un débit supérieur ou égal à 30 litres par minute ; ou
lorsque le gaz contenant de l'oxygène est un gaz mixte d'oxygène et d'un gaz d'équilibre, le module de régulation de débit (201) est configuré pour réguler le gaz contenant de l'oxygène à un débit supérieur ou égal à 30 litres par minute ; et l'interface de source de gaz (101) comprend en outre une interface d'entrée d'oxygène (101a) destinée à fournir de l'oxygène et une interface d'entrée de gaz d'équilibre (101b) destinée à fournir du gaz d'équilibre ; l'appareil de fourniture d'oxygène (102) comprend en outre une branche d'oxygène (204), une branche de gaz d'équilibre (205) et une voie de gaz mixte (206) ; une extrémité d'entrée de gaz de la branche d'oxygène (204) est en communication avec l'interface d'entrée d'oxygène (101a), et de l'oxygène est fourni en entrée à partir de l'interface d'entrée d'oxygène (101a) ; une extrémité d'entrée de gaz de la branche de gaz d'équilibre (205) est en communication avec l'interface d'entrée de gaz d'équilibre (101b), et du gaz d'équilibre est fourni en entrée à partir de l'interface d'entrée de gaz d'équilibre (101b) ; et des extrémités de sortie de la branche d'oxygène (204) et de la branche de gaz d'équilibre (205) sont en communication avec la voie de gaz mixte (206), et l'entrée d'oxygène à partir de la branche d'oxygène (204) et l'entrée de gaz d'équilibre à partir de la branche de gaz d'équilibre (205) sont mélangées et fournies en sortie par le biais de la voie de gaz mixte (206).

2. Machine d'anesthésie (100) selon la revendication 1, comprenant en outre un appareil de régulation de mode (601), lequel répond à une instruction d'opération reçue pour commander l'appareil de fourniture d'oxygène (102) afin de permuter entre au moins deux modes de travail.

3. Machine d'anesthésie (100) selon la revendication 2, comprenant en outre un appareil de surveillance de réglage (602), lequel génère une information d'invite d'alarme lorsqu'une valeur de réglage de débit et/ou de concentration d'oxygène du gaz contenant de l'oxygène fournie par un utilisateur est identifiée comme n'étant pas dans une plage correspondant à un mode de travail en cours.

4. Machine d'anesthésie (100) selon la revendication 1, comprenant en outre un appareil de transmission d'information de travail (603), lequel acquiert une information de travail de l'appareil de fourniture d'oxygène (102) et/ou de l'appareil de respiration d'anesthésie (103) et transmet l'information de travail.

5. Machine d'anesthésie (100) selon la revendication 1, comprenant en outre un appareil d'auto-vérification de défaut (604), lequel surveille une information de défaut de l'appareil de fourniture d'oxygène (102) et/ou de l'appareil de respiration d'anesthésie (103).

6. Machine d'anesthésie (100) selon la revendication 1, comprenant en outre un appareil de régulation de température (605) et/ou un appareil de régulation d'humidité (606), où l'appareil de régulation de température (605) régule une température du gaz contenant de l'oxygène fourni par l'appareil de fourniture d'oxygène (102), et/ou l'appareil de régulation d'humidité (606) régule une humidité du gaz contenant de l'oxygène fourni par l'appareil de fourniture d'oxygène (102).

7. Machine d'anesthésie (100) selon l'une quelconque des revendications 1 à 6, dans laquelle l'appareil de fourniture d'oxygène (102) comprend un module de régulation de concentration (202) destiné à réguler une concentration d'oxygène dans le gaz contenant de l'oxygène, et/ou un module de détection de débit (203) destiné à détecter le débit du gaz contenant de l'oxygène.

8. Machine d'anesthésie (100) selon la revendication 1, dans laquelle le module de délivrance d'anesthésique (401) comprend un mélangeur de gaz (4011) et un évaporateur d'anesthésique (4012) ; le mélangeur de gaz (4011) est relié à l'interface de source de gaz (101) et à l'évaporateur d'anesthésique (4012), et l'oxygène et le gaz d'équilibre sont mélangés puis fournis à l'évaporateur d'anesthésique (4012) ; et l'anesthésique est mélangé avec le gaz fourni par le mélangeur de gaz (4011) puis fourni au circuit de respiration (403) par l'évaporateur d'anesthésique (4012).

9. Système de machine d'anesthésie, comprenant une machine d'anesthésie et un dispositif de fourniture d'oxygène, où la machine d'anesthésie est dotée d'un mécanisme de fixation, et le dispositif de fourniture d'oxygène est fixé de manière amovible à la machine d'anesthésie au moyen du mécanisme de fixation ; où le dispositif de fourniture d'oxygène fournit un gaz contenant de l'oxygène à un patient, le gaz contenant de l'oxygène est de l'oxygène ou un gaz mixte d'oxygène et d'un gaz d'équilibre ; et
lorsque le gaz contenant de l'oxygène est de l'oxygène, le dispositif de fourniture d'oxygène est configuré pour réguler le gaz contenant de l'oxygène à un débit supérieur ou égal à 30 litres par minute ; ou
lorsque le gaz contenant de l'oxygène est un gaz mixte d'oxygène et d'un gaz d'équilibre, le dispositif de fourniture d'oxygène est configuré pour réguler le gaz contenant de l'oxygène à un débit supérieur ou égal à 30 litres par minute ; et le dispositif de fourniture d'oxygène comprend en outre une branche d'oxygène (204), une branche de gaz d'équilibre (205) et une voie de gaz mixte (206) ; une extrémité d'entrée de gaz de la branche d'oxygène (204) est en communication avec une interface d'entrée d'oxygène (101a), et de l'oxygène est fourni en entrée à partir de l'interface d'entrée d'oxygène (101a) ; une extrémité d'entrée de gaz de la branche de gaz d'équilibre (205) est en communication avec une interface d'entrée de gaz d'équilibre (101b), et du gaz d'équilibre est fourni en entrée à partir de l'interface d'entrée de gaz d'équilibre (101b) ; et des extrémités de sortie de la branche d'oxygène (204) et de la branche de gaz d'équilibre (205) sont en communication avec la voie de gaz mixte (206), et l'entrée d'oxygène à partir de la branche d'oxygène (204) et l'entrée de gaz d'équilibre à partir de la branche de gaz d'équilibre (205) sont mélangées et fournies en sortie par le biais de la voie de gaz mixte (206) ;
la machine d'anesthésie est en communication avec le dispositif de fourniture d'oxygène d'une manière filaire ou sans fil ;
l'information de travail de la machine d'anesthésie et du dispositif de fourniture d'oxygène est affichée sur un affichage situé sur l'un d'entre eux, ou un utilisateur est en mesure de commander la machine d'anesthésie et le dispositif de fourniture d'oxygène par le biais d'un écran tactile sur l'un d'entre eux.

10. Système de machine d'anesthésie selon la revendication 9, dans lequel une interface d'entrée de source d'alimentation électrique du dispositif de fourniture d'oxygène est reliée à une interface de source d'alimentation électrique de sortie auxiliaire de la machine d'anesthésie.

11. Système de machine d'anesthésie selon la revendication 9, dans lequel une interface d'entrée de source de gaz du dispositif de fourniture d'oxygène est reliée à une source de gaz externe ou reliée à une source de gaz de la machine d'anesthésie.
